# EUROPEAN PATENT APPLICATION

(11) **EP 3 427 795 A1**
(43) Date of publication of application: **16.01.2019**
(21) Application number: 18181660.4
(22) Date of filing: 04.07.2018
(51) Int. Cl.: A61N 1/372, A61B 5/00, H04L 29/06

(54) **SYSTEM COMPRISING A MEDICAL DEVICE AND AN EXTERNAL DEVICE**

(30) Priority: 11.07.2017 US 201762530859 P
(71) Applicant: BIOTRONIK SE & Co. KG, 12359 Berlin (DE)
(72) Inventor: Müssig, Dirk, West Linn, OR Oregon 97068 (US); Fryer, Alan, Portland, OR Oregon 97202 (US); von Arx, Jeffrey A., Lake Oswego, OR Oregon 97035 (US); Stotts, Larry, Tigard, OR Oregon 97224 (US)
(74) Representative: Keck, Hans-Georg

(57) **Abstract**

The invention refers to a system comprising a medical device (10) and an external device (20), wherein the external device (20) and/or medical device (10) is/are adapted to perform wireless communication with the respective other device. In order to provide for a reliable and low-power method and system providing secure communication between a medical device, in particular an implant, and an external device, the external device (20) comprises a sound signal transmitter (22) and the medical device (10) comprises a sound signal receiver (12), wherein the medical device (10) is adapted such that if the sound signal receiver (12) of the medical device (10) detects a predetermined sound signal sent by the sound signal transmitter (22) of the external device (20), the medical device (10) activates a predetermined operating sequence comprising a wireless data transmission between the medical device (10) and the external device (20). The invention further provides a respective inventive medical device (10), a respective inventive method for operating a medical device (10) using an external device (20), and a respective inventive computer program product for operating a medical device (10) using an external device (20).

## Description

The invention refers a system comprising a medical device and an external device, wherein the external device and/or the medical device is/are adapted to perform wireless communication with the respective other device. The invention further refers to a method for operating a medical device, the respective medical device and a computer program product for operating a medical device.

Implantable, attachable or wearable medical devices are used for monitoring, diagnosis, and treatment of an ever-increasing range of medical conditions. Some non-limiting examples of such medical devices include pacemakers (including leadless pacemakers), implantable loop recorders, implantable cardioverter-defibrillators, deep brain neurostimulators, cochlear implants, gastric implants, and insulin pumps. Medical devices have become increasingly sophisticated over the years and are now commonly equipped with advanced features, such as wireless connectivity. Wireless connectivity can be used for health monitoring, device checkups, manual delivery of therapy, and reprogramming parameters. Healthcare professionals (HCPs) are able to remotely monitor a patient's health and device status through an external device without requiring the patient to visit their office. Patients may also use their own external device to monitor their health and device status. External devices include but are not limited to mobile devices such as mobile phones, smart phones, or tablet computers, or other processing devices such as personal computers or notebook computers. While wireless connectivity in medical devices enables convenient and timely access to medical data, hackers or other adversaries may take advantage of security vulnerabilities to obtain sensitive medical data from the medical devices or even take control of them.

Typically, radio frequency (RF) channels between two wireless devices are secured through the use of cryptographic techniques. Some examples include symmetric or asymmetric key cryptography. However, traditional cryptographic techniques are not directly applicable to medical devices because medical devices must be protected from unauthorized access without deterring or delaying the HCP's access to them, particularly when the patient requires immediate medical assistance. Typical security mechanisms do not address this tension, usually favoring the resistance to adversaries over the need for easy access in emergencies.

Additionally, exchange of a cryptographic key between two parties on an insecure network is a challenging problem. For an implantable medical device using RF telemetry to exchange data though a smart phone for remote monitoring or remote programming, the RF telemetry to smartphone link and the RF telemetry through smart phone to the home monitoring service center link are potentially insecure networks and cryptography keys are needed to secure them. Exchanging cryptography keys with an implantable medical device is difficult. State of the art secrete key exchange on a vulnerable network is typically done with complex computationally expensive algorithms such as the Diffie-Hellman key exchange, but these are extremely challenging to implement in an implantable medical device due to the limited processing power and limited battery capacity in these devices.

The miniaturization of medical devices like monitors and pacemakers puts a higher burden on the power management of the devices. Significant devices shrinkage is only possible if both, the volume of the electronic module as well as the battery is reduced. At the same time the device longevity should not be compromised, which requires that the use of the battery capacity needs to be limited to essential functions as much as possible.

A clinical requirement for medical devices is to record physiological signals (e.g. ECG) when the patient doesn't feel right (e.g. dizziness), which might be an indication for a disturbed heart rhythm. Typically, it is required, that a trigger for such a recording is initiated by the patient by pressing a button on an external device like a smart phone. The smart phone is starting a communication session (e.g. via -telemetry Bluetooth Low Energy (BLE)) with the medical device trying to initiate the recording. In order to allow for such a communication session the medical device needs to listen on a (at least) periodic interval for commands from the external device. -Wireless communications like BLE are significantly contributing to the power consumption of the implanted medical device and therefore even the periodic short time periodic listening is reducing the device longevity significantly.

Document US 2016/0250490 A1 describes a method for authorized telemetry between an implant within a patient's body and an external device (e.g. a smartphone). This document discloses that the method includes comparing first electronic information with second electronic information. The method further includes determining whether a defined level of correlation exists between the first electronic information and the second electronic information, and initiating a telemetry session between the external device and the implant based on a determination that the defined level of correlation exists between the first electronic information and the second electronic information. For example, the first electronic information is indicative of a speech signal recorded by the external device and the second electronic information is indicative of the vibration information internal to the body and detected by the implant. The predetermined correlation is only achieved if the speech signal was produced by the patient wearing the implant thereby causing the vibration information and the external device directly receives the speech signal.

Document WO2016/133813 A1 discloses generating a secure communication side channel for wireless communication using vibration signals between an external device and an implant. The document also describes usage of the vibration signals as wakeup-call for the implant. It also discloses exchange of a cryptographic key in form of a vibration signal between the implant and the external device.

Therefore it is an object of the invention to provide for a reliable and low-power method and system providing secure communication between a medical device, in particular an implant, and an external device.

The above problem is solved by the system defined in claim 1.

In particular, the system comprises a medical device and an external device, wherein the external device and/or medical device is/are adapted to perform wireless communication with the respective other device,
wherein the external device comprises a sound signal transmitter and the medical device comprises a sound signal receiver,
wherein the medical device is adapted such that if the sound signal receiver of the medical device detects a predetermined sound signal sent by the sound signal transmitter of the external device, the medical device activates a predetermined operating sequence comprising a wireless data transmission between the medical device and the external device. With other words: The detection of the predetermined sound signal of the external device wakes up the medical device and triggers execution of the predetermined operating sequence. The predetermined operating sequence may include an operating sequence section provided by the external device.

According to the invention, the medical device is a wearable, implantable device and/or attachable device to the patient's body. For example, the medical device may be an active or passive implant which is capable of measuring cardiac parameters, as for instance a cardiac pacemaker, and implantable loop recorder, and implantable cardioverter-defibrillator, a leadless pacemaker, a neurostimulator for the spinal cord or the vagus nerve, a watch monitoring body parameters of a patient or the other medical devices mentioned above. The external device is a device external of the patient's body, for example a personal computer, a smart phone, a tablet computer or another remote device comprising a microprocessor. The external device is accessible by the patient and/or the HCP.

The sound signal transmitter of the external device is for example a loudspeaker, a piezoelectric transducer, a mechanical vibrator (such as a smart phone's vibration alert), or any other well known transducer for producing sound.

The sound signal receiver of the medical device is, for example, a microphone, an accelerometer, or a piezoelectric transducer, a hydrophone, or another transducer.

According to the invention, the sound signal comprises an infrasound signal, an ultrasound signal, a hypersound signal, or a sound signal comprising the frequency range hearable for the human being. In the preferred embodiment the sound signal is in the range of 40 Hz to 10 kHz. The sound signal is a signal that propagates as a mechanical wave of pressure and displacement through a transmission medium such as air, water or bodily tissue.

Wireless data transmission comprises transmission over the air (without wire) using electromagnetic waves, for example Bluetooth, WLAN, ZigBee, NFC, Wibree or WiMAX in the radio frequency region, or IrDA or free-space optical communication (FSO) in the infrared or optical frequency region.

According to the invention, both, the sound signal transmission and wireless data transmission between the medical device and the external device may be either one-directional or bi-directional data/signal transmission.

The inventors recognized that sound waves (acoustic waves) travel very well through the body of a patient. The acoustic impedance of various body tissues as well as that of air is given in the following table containing acoustic impedance of various propagation media.

| **Propagation Medium** | **Acoustic Impedance (kg·m⁻²s⁻¹·10⁻⁶)** |
|---|---|
| Water | 1.50 |
| Bone (average) | 6.00 |
| Blood | 1.59 |
| Muscle (average) | 1.70 |
| Soft Tissue (average) | 1.58 |
| Fat | 1.38 |
| Air | 0.000429 |

Accordingly, the inventive system provides an acoustic secure side channel for sharing a secret key or for triggering recording of data, for example of at least one physiologic parameter by the medical device.

Sound signal receivers like accelerometers in medical devices are always on and draw only a few 100 nA of current from the battery and are, for example in pacemakers, primarily used for rate responsive pacing. These physical accelerometers are typically sensitive up to a kHz or more in frequency, although they are amplified and sampled typically in range of a few 10s of Hz. The loudspeakers of smartphones operate from about 40 Hz to 10 KHz. This means that it is reasonable to assume about 10 times the current will be needed to continuously monitor for a signal. This means roughly 1µA of current draw will be required to continuously monitor for a wake up signal with the accelerometer. However, it is not necessary to continuously monitor for the signal. Detection can be duty cycled down to one or two monitoring windows of a few tens of ms each second, which will reduce the average current draw by a factor of 10 or more. Therefore 100 nA or less average current draw is likely achievable.

This must be compared to the current draw of the prior art, which would be using for example the BLE transceiver in the external device, for example a smartphone, to poll for signal. These transceivers typically draw 12 mA or more of current in receive mode, and they cannot be quickly turned off and cycled back on for duty cycling. As can be seen from this analysis, the acoustic wakeup can potentially draw about 100,000 times less current than the prior art alternative. Once the implant is woken up via the sound signal according to the invention, then the medical device can establish a higher power RF link (such as BLE) with the external device.

In one embodiment the external device comprises a coupling element which transmits or couples the sound signals produced by the sound signal transmitter of the external device directly to the patient's body if placed at the surface of the patient's body such that the patient's body is in direct contact with the coupling element. This is a preferred embodiment because the impedance of air is four orders of magnitude away from the impedances of body tissues. The huge impedance mismatch between a person's body and the surrounding air means that the body makes a great acoustic communication channel, and yet it is well isolated from the air surrounding the body. The acoustic receiver of the medical device communicates acoustically with, for example, a smart phone held in intimate contact by the patient, but that is not sensitive to the many orders of magnitude smaller signals from sources which are not in contact with the human body. The smart phone held in intimate contact, for example with the chest of the patient or with the hand of the patient, can generate enough pressure to be reasonably sensed in the body by implantable medical devices (and vice versa, signals generated by the implant can be reasonable sensed by the smart phone in intimate contact with tissue). However, phones held by people nearby the patient will in most cases not generate enough of a signal to be sensed by the implant. This is because acoustic waves are reflected at (rather than transmitted through) boundaries of impedance mismatch. The huge acoustic impedance mismatch between a body and air effectively filters out all signals except one made by a phone in intimate physical contact with the patient.

In one embodiment the coupling element is the full housing or a section of the housing of the external device, for example one or a plurality of recess(es) for a fingertip. In the preferred embodiment the coupling element is the case of the smart phone, which the patient is instructed to press across his/her chest during acoustic communication. The smart phone should be placed as close to directly over the implant as possible to ensure good signal coupling.

Accordingly, in one embodiment the predetermined operating sequence comprises data recording by the medical device, for example detecting the heart frequency, recording an electrocardiogram (ECG), recording patient activity, etc.

In an embodiment of the invention, medical device comprises a communication transceiver for performing wireless communication, e.g. an RF transceiver. The predetermined operating sequence comprises transferring the communication transceiver of the medical device from an inactive state to an active state, i.e. to 'wake up' the communication transceiver. After 'wake up', the communication transceiver may acknowledge that the sound signal was received by sending a signal to the external device, and listen for a command on a communication channel, e.g. an RF communication channel, from the external device. In this way the external device can send further commands that are not predetermined.

In one embodiment the operating sequence comprises data recording by the medical device during a predetermined time interval or as long as a predetermined number of data is recorded. According to the invention data recording means measuring physiological data and/or determining physiological data of the patient by a measuring unit of the medical device and saving the data within a data storage unit of the medical device, wherein the data storage unit is coupled to the measuring unit of the medical device. In one embodiment the recorded data are transmitted to the external device by wireless communication / data transmission.

According to an embodiment of the present invention, if the patient experiences an episode that is indicative for a harmful or pathologic medical condition, the patient launches for example an application on the patient's smart phone for activation a predetermined operating sequence that measures a suitable physiological parameter or a plurality of suitable physiological parameters after authorization using the sound signal produced by the external device.

In another embodiment, data transmission and/or the command on a communication channel comprises key exchange between the medical device and the external device.

In another embodiment one key is transmitted from the external device to the medical device by the sound signal sent via the sound signal transmitter of the external device. For that the sound signal transmitter is adapted to send the key with the sound signal, for example using signal modulation techniques. The acoustic waves may be modulated with On/Off Keying (OOK), Amplitude Shift Keying (ASK), Frequency Shift Keying (FSK), Quadrature Phase Shift Keying (QPSK), Pulse position encoding, or some other modulation technique. Accordingly, the sound signal receiver of the medical device is adapted to demodulate the modulated sound signals. The encryption key may be either randomly generated by the external device or obtained from a pre-defined source, for example from a Home Monitoring Service Center, or from a storage unit of the external device.

In another embodiment the sound signal sent by the sound signal transmitter of the external device comprises at least one parity bit and/or at least one cyclic redundancy bit, preferably modulated with the sound signal. The at least one parity bit and/or the at least one cyclic redundancy bit is checked by the sound signal receiver of the medical device after demodulation of the sound signal in order to detect transmission errors. If this check reveals a transmission error the control unit of the medical device assumes that the received sound signal is not the predetermined sound signal.

In another embodiment the external device comprises a trigger operable by the patient or the HCP, wherein the trigger is connected with the sound signal transmitter in order to initiate sending the wake-up sound signal. The trigger may be realized for example by a switch or the button provided by the external device, for example at a touchscreen of the external device provided by an application.

The above-mentioned problem is also solved with the advantages described above by a medical device as defined in claim 11.

The inventive medical device is, in particular, adapted to perform wireless communication with an external device, wherein the medical device comprises a sound signal receiver and is adapted such that if the sound signal receiver of the medical device detects a predetermined sound signal sent by the sound signal transmitter of the external device, the medical device activates a predetermined operating sequence comprising a wireless data transmission between the medical device and the external device.

The above-mentioned problem is also solved by a method for operating a medical device using an external device comprising the steps as defined in claim 12.

In particular, the method comprises the following steps:
sending a predetermined sound signal by a sound signal transmitter of the external device, detecting sound signals by a sound signal receiver of the medical device,
activating and performing a predetermined operating sequence by the medical device comprising a wireless data transmission between the medical device and the external device if the predetermined sound signal was detected by the sound signal receiver of the medical device.

The inventive method has the advantages as explained with regard to the inventive system.

As indicated above, in one embodiment the external device is positioned at the surface of the patient's body prior and/or during sending the predetermined sound signal, for example at the chest or at the hand of the patient grabbed by the fingers.

In another embodiment, the performing of the predetermined operating sequence comprises data recording by the medical device.

In another embodiment, the performing of the predetermined operating sequence and/or the command on a communication channel comprises key exchange between the medical device and the external device.

In another embodiment, the preforming of the predetermined operating sequence comprises waking up the RF transceiver in the implant and initiating an RF communication session.

In a further embodiment, the sound signal transmitter of the external device sends the predetermined sound signal only if sending was triggered by the patient or the HCP using a trigger operable by the patient or the HCP, wherein the trigger is connected with the sound signal transmitter.

The above-mentioned task is further solved by a computer program product for operating a medical device using an external device with program code means for execution of a computer program after implementation within the medical device and/or the external device, i.e. the respective microprocessor of the medical device and/or the external device, wherein the program code means are provided to execute the above described method.

A full and enabling disclosure of the present invention, including the best mode thereof, directed to one of ordinary skill in the art is set forth in the following specification of the preferred embodiments. Thereby, further features and advantages are presented that are part of the present invention independently of the features mentioned in the dependent claims.

The specification refers to the accompanying Figures showing schematically
- Fig. 1: an inventive system comprising a patient with a medical device and an external device in a front view, and
- Fig. 2: a flow chart of a first embodiment of the inventive method.

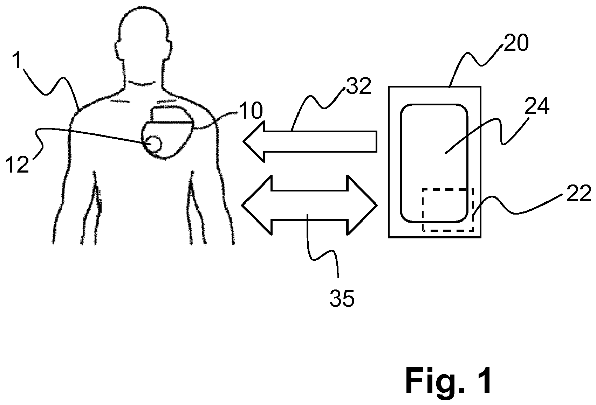

Figure 1 shows a patient 1 with an implanted medical device such as a pacemaker 10 and an external device such as a smartphone 20.

The pacemaker 10 comprises an accelerometer 12 as a sound signal receiver and means for wireless communication as well as detection means for electrical signals of the heart, e.g. implanted electrodes.

The smart phone 20 comprises an application for the pacemaker 10 which is displayed on the display/touchscreen 24. The smart phone 20 further comprises a loudspeaker 22 which may be controlled by the application for the pacemaker 10.

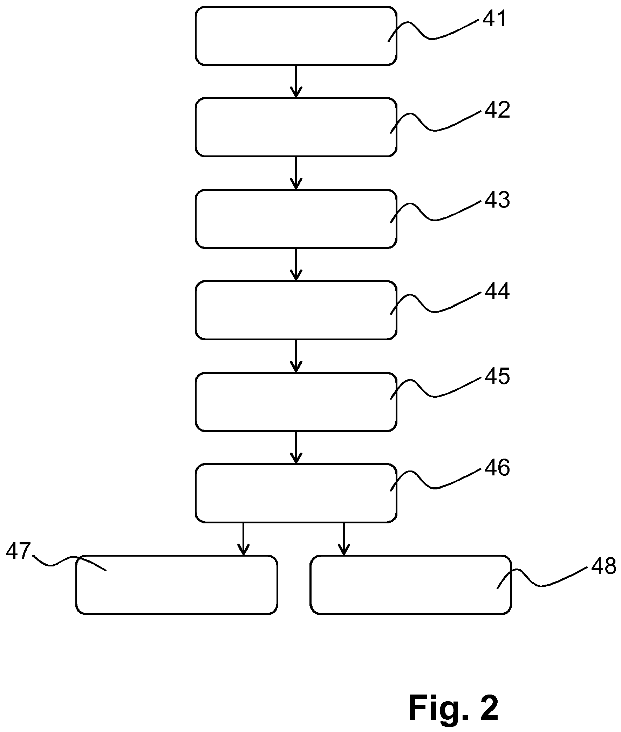

A first embodiment of the inventive method works as follows. If the patient 1 with the pacemaker 10 feels symptomatic (see step 41 in figure 2) the patient 1 launches the application for the pacemaker 10 on his/her smart phone 20 (step 42 in figure 2). Now, the application instructs the patient 1 to place a coupling element of his/her smart phone 20 on his/her chest (see step 43 in figure 2). The coupling element may be provided by a section of the housing of smart phone connected to the loudspeaker 22 of the smart phone. Subsequently, the smart phone 20 sends periodic sound signals (depicted by arrow 32) at a either a fixed frequency and duty cycle, or a modulated frequency and/or duty cycle (step 44 in figure 2), which sound signals directly couple into the patient's body and propagate through the body of the patient 1 and into the direction of the pacemaker 10. In the prefered embodiment the signal is modulated. This allows the signal to contain data, which in one embodiment is used to reduce the possibility of noise causing a trigger (e.g. a multi-bit wakeup signal), and in a different embodiment it allows the acoustic signal to transmit a cypher key. At the same time or subsequently, the smart phone 20 turns on its BLE, Bluetooth, MICS, ZigBee, Wifi, or other RF communication system and starts looking for signals from the pacemaker 10 (step 45 in figure 2). Now, the accelerometer 12 of the pacemaker 10 detects the sound signals from the smart phone 20, transduces the sound signals into respective electric signals. The pacemaker circuitry then compares the electric signals with an electric signal stored within a storage unit of the pacemaker 10 (step 46 of figure 2). If the electric signals transmitted from the detected sound signals correspond to the stored electric signal a control unit of the pacemaker 10 assumes that a predetermined wake-up sound signal is received by the accelerometer 12. The pacemaker then activates a predetermined operation sequence and, for example confirms that the communication path to the pacemaker 10 is established and that the operation sequence is started by a signal provided by Bluetooth communication (step 47 of figure 2). Wireless Bluetooth communication is depicted in figure 1 by arrow 35. If the electric signals transmitted from the detected sound signals do not correspond with the stored electric signal, the control unit of the pacemaker 10 does not activate the predetermined operation sequence. The accelerometer 12 then continues receiving incoming sound signals (step 48 of figure 2). If the RF communication session 35 fails to initiate for any reason, the accelerometer 12 then continues receiving incoming sound signals. In one embodiment the sound detection of the accelerometer 12 is duty cycled to save power in the implant.

Duty cycling of the accelerometer achieves a reasonable longevity for the implant. In a preferred embodiment of the invention, the accelerometer is set on at a low sampling rate to detect activity, e.g. 20 Hz, but is periodically sampled at a higher sample rate, e.g. 600 Hz for detecting vibrations. In an embodiment, sampling for detecting vibrations is performed in a sampling time window, wherein the higher sample rate may be applied continuously or phased within the sampling time window. For example, the sampling time window may be one second, wherein sampling via the higher sampling rate occurs twice within that time window.

The predetermined operation sequence may comprise measurement of electric heart signals by an electrode connected with the pacemaker 10 during a predetermined time period or as long as a predetermined number of heart signal measurement values are measured. The measured heart signals are transmitted to the smart phone 20 for example via Bluetooth communication (see arrow 35). The application of the smart phone 20 may initiate a measurement sequence which may be stored within the storage unit of the smart phone 20 or which may be provided by patient's or HCP's respective input using the touchscreen 24 of the smart phone 20. Alternatively, the measurement sequence may be provided by the pacemaker 10 and stored within the storage unit of the pacemaker 10.

In a second embodiment of the inventive method, a modulated sound signal is produced by the loudspeaker 22 of the smart phone 20 in order to wake-up the pacemaker 10.

Accelerometers in most pacemakers and ICDs are well suited for picking up sound signals generated from phone's loudspeaker (as long as the circuitry around the accelerometer is designed to sample the accelerometer fast enough, and the filters are designed to pass this higher frequency). These signals generally are in the 300 Hz to 3,000 kHz range, and this can easily be used to generate a data stream of 100 bits per second. At these data rates, a 8-bit wakeup signal can be transmitted in 100 ms, and a 128 bit key can be transmitted in about 1.3 s.

The sound signal sent by the loudspeaker 22 of the smartphone 20 may contain parity bits and/or cyclic redundancy bits for error detection. Upon receiving the transmitted sound signal (see arrow 32), the accelerometer 12 demodulates the acoustic signal to recover the data payload, and then the control unit of the pacemaker 10 checks for transmission errors using the parity bits and/or the cyclic redundancy bits. Assuming there are no errors, the pacemaker 10 sends an acknowledgement signal to the smart phone 20, for example via wireless Bluetooth communication (see arrow 35).

**The acoustic** link comprising the wake up sound signal (see arrow 32) may be unidirectional from the smart phone 20 to the pacemaker 10. In one embodiment the acoustic link is used to send a key from the smart phone application to the implanted device, but not used to send the acknowledgement back to the smart phone. For example, an RF communication link such as Bluetooth low energy (BLE) is used to send the acknowledgement from the implant back to the smart phone. The acknowledgement can be sent encrypted with the new shared key. In another embodiment, the acoustic link is bi-directional, and the acknowledgement is sent back using a sound signal. In this case the pacemaker 10 has a loudspeaker, hydrophone, or a piezo electric element which produces the acknowledgement sound signal. In a third embodiment the key exchange is facilitated using the wireless communication, for example BLE communication, activated by the sound signal(s). For example, the Diffie-Hellman Key Exchange is a well-known public private key exchange technique.

After key exchange, for example, the working parameters of the pacemaker 10, for example the standard pacing frequency, the pacing amplitude, the sensitivity of cardiac signal sensing, the rate responsive sensitivity of the accelerometer, the pacing mode, enabling MRI mode, etc. may be changed by the HCP using the secure communication channel. In one embodiment some adjustable parameters are not allowed to be adjusted by the patient's smart phone, such as pacing rate, pacing amplitude, pacing mode, etc. These can only be adjusted by a HCP physician's programmer for safety reasons.

In one embodiment the phone may instruct the patient to hold the phone directly over his/her chest when triggered by the patient. Holding the phone directly in contact with the patient's chest will help maximize coupling of vibrations into the body and will minimize the distance the sound signals need to travel through the body. The phone's instructions to the patient to place phone over chest may be audio (through the phone's speaker), visual or both. The visual display may include an illustration of optimal phone placement over the chest.

A second embodiment of the inventive method works as follows (this illustrates just one embodiment):
At first the patient 1 launches a pacemaker smart phone application on his/her smart phone 20. Then the application checks to see if the smart phone 20 has an encryption/authentication key stored in its storage unit. If it does not have a key stored, or if it has a key stored but the key is expired, then the smart phone 20 prompts the patient to hold the phone on his/her chest for key exchange. The smart phone displays on its screen 24 a diagram of where and how to hold the smart phone 20. The patient is instructed to hold the phone there until sending of the sound signal (see arrow 32) stops.

After a pause to allow the patient 1 to position the smart phone 20 (i.e. 5 seconds), the smart phone 20 uses its loudspeaker 22 to sending out a sound signal (see arrow 32) containing an encryption key via modulating the sound signal. In one embodiment the modulation is on/off shift keying modulation or OOK modulation. In OOK modulation the sound signal is turned on and off in sequence to transmit a logical 1 or 0. The encryption key may either be randomly generated by the smart phone 20 (for securing the communication with the smart phone 20) or obtained, for example, from the Home Monitoring Service Center over a GSM modem of the smart phone using 20 using public/private encryption techniques such as Diffie-Hellman or Rivest, Shamir and Adleman (RSA, see e.g. US 4,200,770). Then, the accelerometer 12 of the pacemaker 10 detects the sound signals from the smart phone 20 and demodulates the signal to obtain the data packet. The patient 1 continues to hold the smart phone 20 on his/her chest for as long as it takes to transmit the encryption key. Sound signals generated from phone's loudspeaker will generally are in the 300 Hz to 3,000 kHz range, and this can easily be used to generate a data stream of 100 bits per second. At these data rates, a 8-bit wakeup signal can be transmitted in 100 ms, and a 128 bit key can be transmitted in about 1.3 s.

In the next step, the pacemaker 10 checks the received data packet for errors by preforming a cyclic redundancy check. Assuming that the key was successfully received, the pacemaker 10 then turns on its BLE subsystem, uses the new, transmitted encryption key to secure the BLE link, and sends an acknowledgement signal using the BLE subsystem to the smart phone 20. At this point the key exchange is complete.

In case the encryption key was not successfully received by the pacemaker 10, then no acknowledgement signal is sent to the smart phone 20, and the smart phone 20 retries sending the encryption key across the acoustic link until it either receives an acknowledgement, or it times out and prompts the patient 1 to re-position the smart phone 20 on the chest and try again.

According to the invention described above, no additional hardware is required to wake-up the pacemaker 10 to trigger different reactions of the pacemaker 10 or to allow for secure key exchange between the pacemaker 10 and the smart phone 20. In addition the pacemaker 10 can keep higher energy demanding function in active and therefore extend the longevity of the pacemaker 10. Further, the inventive method also provides a means for documentation that a patient or HCP gives consent by requiring the action using the smart phone application. This effectively means that the patient or HCP authenticates that they feel that communication is legitimate. Since the smart phone must be in intimate contact with the patient in order for the acoustic waves to couple to the implant, only the patient or a care giver in physical contact with the patient can successfully use the smart phone application.

### Reference numbers

- 1: patient
- 10: pacemaker
- 12: accelerometer
- 20: smart phone
- 22: loudspeaker
- 24: display/touch screen
- 32: arrow showing acoustic link
- 35: arrow showing RF link (e.g. BLE)
- 41, 42, 43, 44, 45, 46, 47, 48: steps of an embodiment of the inventive method

## Claims

1. A system comprising a medical device (10) and an external device (20), wherein the external device (20) and/or medical device (10) is/are adapted to perform wireless communication with the respective other device,
wherein the external device (20) comprises a sound signal transmitter (22) and the medical device (10) comprises a sound signal receiver (12),
wherein the medical device (10) is adapted such that if the sound signal receiver (12) of the medical device (10) detects a predetermined sound signal sent by the sound signal transmitter (22) of the external device (20), the medical device (10) activates a predetermined operating sequence comprising a wireless data transmission between the medical device (10) and the external device (20).

2. A system according to claim 1, wherein the sound signal comprises a vibration, wherein the sound signal transmitter (22) comprises a vibration transmitter and the sound signal receiver (12) comprises a vibration receiver.

3. A system according to one of the preceding claims, wherein the medical device (10) comprises a communication transceiver for performing wireless communication, wherein the predetermined operating sequence comprises transferring the communication transceiver of the medical device from an inactive state to an active state.

4. A system according to claim 3, wherein the communication transceiver is configured to
- send a signal to the external device (20) for acknowledging that the sound signal from the external device (20) was received,
- listen for a command on a communication channel from the external device (20).

5. The system according to one of the preceding claims, wherein the sound signal comprises an infrasound signal, an ultrasound signal, a hypersound signal or a sound signal comprising the frequency range hearable for the human being.

6. The system according to one of the preceding claims, wherein the external device (20) comprises a coupling element which transmits the sound signals produced by the sound signal transmitter (22) to the patient's body if placed at the surface of the patient's body.

7. The system according to any of the previous claims, wherein the data transmission and/or the command on a communication channel comprises key exchange between the medical device (10) and the external device (20).

8. The system according to any of the previous claims, wherein the external device (20) comprises a trigger operable by the patient (1) or the HCP, wherein the trigger is connected with the sound signal transmitter (22).

9. A method for operating a medical device (10) using an external device (20) comprising the following steps,
sending a predetermined sound signal by a sound signal transmitter (22) of the external device (20),
detecting sound signals by a sound signal receiver (12) of the medical device (10), activating and performing a predetermined operating sequence by the medical device (10) comprising a wireless data transmission between the medical device (10) and the external device (20) if the predetermined sound signal was detected by the sound signal receiver (12) of the medical device (10).

10. The method according to claim 9, wherein the predetermined operating sequence comprises transferring a communication transceiver of the medical device from an inactive state to an active state.

11. The method according to claim 10, further comprising the steps,
- sending a signal to the external device (20) for acknowledging that the sound signal from the external device (20) was received,
- listening for a command on a communication channel from the external device (20).

12. The method according to at least one of the claims 9 to 11, wherein the external device (20) is positioned at the surface of the patient's body prior and/or during sending the predetermined sound signal.

13. The method according to at least one of the claims 9 to 12, wherein the performing of the predetermined operating sequence and / or the command on a communication channel comprises key exchange between the medical device (10) and the external device (20).

14. The method according to at least one of the claims 9 to 13, wherein one key is transmitted from the external device (20) to the medical device (10) via the sound signal sent by the sound signal transmitter (22) of the external device (20).

15. The method according to at least one of the claims 9 to 14, wherein the sound signal transmitter (22) of the external device (20) sends the predetermined sound signal only if sending was triggered by the patient (1) or the HCP using a trigger operable by the patient (1) or the HCP, wherein the trigger is connected with the sound signal transmitter (22).
